# EUROPEAN PATENT SPECIFICATION

(11) **EP 2 558 059 B1**
(45) Date of publication and mention of the grant of the patent: **12.06.2019**
(21) Application number: 11715834.5
(22) Date of filing: 13.04.2011
(51) Int. Cl.: A61K 8/26, A61K 8/44, A61K 8/58, A61K 8/36, A61K 8/02, A61Q 17/04

(54) **COMPOSITIONS AND METHODS FOR SPF ENHANCEMENT BY HIGH CONCENTRATION COSMETIC POWDER FORMULATIONS**
ZUSAMMENSETZUNGEN UND VERFAHREN FÜR SPF-VERSTÄRKUNG MIT HOCHKONZENTRIERTEN KOSMETIKPUDERFORMULIERUNGEN
COMPOSITIONS ET PROCÉDÉS PERMETTANT D'AMÉLIORER LE FACTEUR DE PROTECTION SOLAIRE GRÂCE À DES FORMULES DE POUDRE COSMÉTIQUE À CONCENTRATION ÉLEVÉE

(30) Priority: 13.04.2010 US 323678 P
(43) Date of publication of application: 20.02.2013
(73) Proprietor: U.S. Cosmetics Corporation, Dayville, CT 06241 (US)
(72) Inventor: KISHIDA, Shigeru, Storrs CT 06268 (US); KAWASAKI, Yoshiaki, Woodstock CT 06281 (US); YAKUPCIN, Ronald, Matamoras PA 18336 (US)
(74) Representative: Beck Greener LLP
(86) International application number: PCT/US2011/032236
(87) International publication number: WO 2011/130358

(56) References cited:
- EP-A1- 0 761 201
- EP-A2- 1 371 357
- WO-A1-02/062310
- WO-A1-03/045345
- WO-A2-2010/093573
- WO-A2-2010/148180
- US-A1- 2008 299 158
- US-B1- 6 482 441
- ODOM I E: "SMECTITE CLAY MINERALS: PROPERTIES AND USES", PHILOSOPHICAL TRANSACTIONS OF THE ROYAL SOCIETY OF LONDON,SERIES A; MATHEMATICAL AND PHYSICAL SCIENCES, ROYAL SOCIETY, LONDON, GB, vol. 311, no. 1517, 14 June 1983 (1983-06-14), pages 391-409, XP002594660, ISSN: 0080-4614, DOI: 10.1098/RSTA.1984.0036
- Anonymous: "Smectite Group: Smectite Group mineral information and data.", , 23 October 2016 (2016-10-23), pages 1-4, XP055314984, Retrieved from the Internet: URL:http://www.mindat.org/min-11119.html [retrieved on 2016-10-28]

## Description

### FIELD OF THE INVENTION

The present disclosure relates generally to cosmetic formulations wherein cosmetic powder materials potentiate the UV protection factor provided by an organic sunscreen.

### BACKGROUND

Many lotion-type sunscreens are currently available on the market. Typically, sunscreen formulas are water-in-oil (W/O) or oil-in-water (O/W) emulsions or are anhydrous systems. In order to obtain a high sun protection factor (SPF) and particularly a high protection factor relative to UV-A radiation (PFA), sunscreen formulations typically incorporate extensive amounts of oil-based, UV-active materials. The use of large amounts of oil-based, UV-actives causes the texture of the resulting sunscreens to be oily, greasy, tacky, and somewhat opaque. Oils are also undesirable because they may enhance the transdermal permeation of other formulation ingredients including ingredients for which transdermal administration may be inappropriate. In addition to these undesirable properties, the high loading of oil-based UV-actives often causes adverse skin reactions in sensitive individuals.

Commercial sunscreens are typically formulated to yield about 1 to 2 SPF units per weight percent (wt%) UV-active ingredient. For example, typical SPF 20 sunscreen formulations contain approximately 13% UV-active materials. It is often desirable to formulate sunscreen with much higher SPF ratings. To formulate sunscreens at the higher SPF rating requires corresponding increases in the concentration of oil-based, organic UV-actives.

It is desirable to formulate sunscreens with increasingly high SPF values to confer higher levels of protection. However, the current formulation metric implies higher degrees of unwanted side-effects. If organic UV absorbers are used in formulations at the lowest possible level, tactile issues and safety concerns would be ameliorated. Also, production costs would be lower as lesser amounts of raw materials are used. Therefore, there exists a need to formulate sunscreens having lower amounts of organic UV-actives.

Other objects and advantages will become apparent from the following disclosure.

US6482441 discloses a surface-treated powder for cosmetics which may have UV light shielding performance and which has good dispersibility.

WO03045345 discloses cosmetic compositions containing inorganic fillers and thus exhibiting particular effects in terms of UV behaviour with or without organic UV filters.

WO02062310 discloses cosmetic compositions using particulate-based thickeners such as smectite clays, which may be co-dispersed with particulate UVR-filters.

### SUMMARY OF INVENTION

The present disclosure relates to an SPF-boosting composition having an SPF-index of at least 3.0, comprising organic UV-protective agents and a cosmetic powder, wherein a given level of UV protection is achieved at a lower concentration of organic UV-protective agents than is possible in the absence of the cosmetic powder, the composition comprising: up to 30 wt% of a non-volatile oil comprising at least two organic UV-active materials, at least 30 wt% of an aqueous phase; and from 10 wt% to 25 wt% of the cosmetic powder, wherein said cosmetic powder is coated with at least one surface-active agent chemically-immobilized to a surface thereof, wherein the cosmetic powder selected from the group consisting of mica, aluminum calcium sodium silicate, talc, kaolin, bismuth oxychloride and mixtures thereof; and wherein the at least one surface active agent is selected from any of triethoxycaprylylsilane, methicone, dimethicone, or triethoxycaprylylsilane (and) aluminum myristate (and) disodium stearoyl glutamate. According to aspects of the disclosure, the composition has an SPF Index of at least 4.0, more preferably of at least 5.0. According to aspects of the disclosure, the compositionhas an SPF Index of at least 6.0. According to aspects of the disclosure, the composition has an SPF Index of at least 8.0. According to aspects of the disclosure, the composition has an SPF Index of at least 10.0.

The organic UV-active is any organic sunscreen which absorbs, blocks, or otherwise mitigates ultraviolet radiation.

According to an aspect of the disclosure, the invention excludes volatile silicone oils.

The composition comprises a cosmetic powder material present at a concentration that potentiates the UV-blocking activity of an organic UV-active material. According to an aspect of the disclosure, the cosmetic powder material is present in at least an inflection concentration.

Still other aspects and advantages of the present invention will become readily apparent by those skilled in the art from the following detailed description, wherein it is shown and described preferred embodiments of the invention, simply by way of illustration of the best mode contemplated of carrying out the invention. Accordingly, the description is to be regarded as illustrative in nature and not as restrictive.

### BRIEF DESCRIPTION OF DRAWINGS

The invention is best understood from the following detailed description when read in connection with the accompanying drawing. Included in the drawing are the following figures:
Figure 1 shows the sigmoid dependence of the value of SPF on the concentration of surface-modified cosmetic powder materials;
Figure 2 shows the sigmoid dependence of the value *of in vitro* SPF on the concentration of kaolin treated with triethoxycaprylylsilane (and) aluminum dimyristate (and) disodium stearoyl glutamate;
Figure 3 shows the sigmoid dependence of the value *of in vitro* PFA on the concentration of kaolin treated with triethoxycaprylylsilane (and) aluminum dimyristate (and) disodium stearoyl glutamate;
Figure 4 shows the sigmoid dependence *of in vitro* SPF on the concentration of untreated kaolin; and,

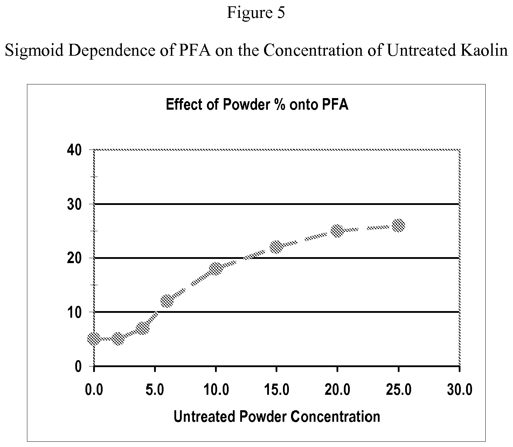

Figure 5 shows the sigmoid dependence *of in vitro* PFA on the concentration of untreated kaolin;

It is to be noted, however, that the appended drawings illustrate only typical embodiments of this invention and are therefore not to be considered limiting of its scope, for the invention may admit to other equally effective embodiments.

### DETAILED DESCRIPTION OF A PREFERRED EMBODIMENT

Reference is made to the figures to illustrate selected embodiments and preferred modes of carrying out the invention. It is to be understood that the invention is not hereby limited to those aspects depicted in the figures.

The composition of the present invention comprises up to 30 wt% of a non-volatile oil. The non-volatile oil comprises at least two organic, UV-active materials that function as UV-protective agents ("sun blocks"). Two or more organic, UV-actives are used to provide a wide spectrum of protection in the UV region. For example, a combination of at least one UV protecting agent that mainly provides protection against UVA light, and at least one UV protecting agent that mainly provides protection against UVB light, may be used.

A wide variety of conventional UV protecting agents are suitable for use herein. Non-limiting exemplary organic, UV-actives include: 2-ethylhexyl-p-methoxycinnamate (commercially available as PARSOL MCX), butylmethoxydibenzoyl-methane, 2-hydroxy-4-methoxybenzo-phenone, 2-phenylbenzimidazole-5-sulfonic acid, octyldimethyl-p-aminobenzoic acid, octocrylene, 2-ethylhexyl N,N-dimethyl-p-aminobenzoate, p-aminobenzoic acid, 2-phenylbenzimidazole-5-sulfonic acid, octocrylene (Parsol 340, DSM), oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, Eusolex.TM. 6300, avobenzone (Parsol 1789, DSM), avobenzone, PABA, octyldimethyl-PABA, Phenylbenzimidazole sulfonic acid, Cinoxate, Dioxybenzone (Benzophenone-8), Oxybenzone (Benzophenone-3), Homosalate, Menthyl anthranilate, Octisalate, Sulisobenzone, Trolamine salicylate, Terephthalylidene Dicamphor Sulfonic Acid, 4-Methylbenzylidene camphor, Methylene Bis-Benzotriazolyl Tetramethylbutylphenol, Bis-ethylhexyloxyphenol methoxyphenol triazine, bisimidazylate, Drometrizole Trisiloxane, Octyl triazone, Diethylamino Hydroxybenzoyl Hexyl Benzoate, Iscotrizinol, Polysilicone-15, Amiloxate, Ethylhexyl Dimethoxybenzylidene Dioxoimidazolidine Propionate, and mixtures thereof. Preferred organic UV active materials are octinoxate, octocrylene, avobenzone, PABA, octyldimethyl-PABA, Phenylbenzimidazole sulfonic acid, Cinoxate, Dioxybenzone (Benzophenone-8), Oxybenzone (Benzophenone-3), Homosalate, Menthyl anthranilate, Octisalate, Sulisobenzone, Trolamine salicylate, Terephthalylidene Dicamphor Sulfonic Acid, 4-Methylbenzylidene camphor, Methylene Bis-Benzotriazolyl Tetramethylbutylphenol, Bis-ethylhexyloxyphenol methoxyphenol triazine, bisimidazylate, Drometrizole Trisiloxane, Octyl triazone, Diethylamino Hydroxybenzoyl Hexyl Benzoate, Iscotrizinol, Polysilicone-15, Amiloxate, Ethylhexyl Dimethoxybenzylidene Dioxoimidazolidine Propionate, and mixtures thereof.

In addition to a UV-active, the non-volatile oil may comprise an ancillary oil which may be a solvent for one or more of the UV-active oils. The ancillary oil may provide desirable cosmetic properties such as emolliency and a good "skin feel." A preferred, but non-limiting ancillary oil is isopropyl myristate.

Non-volatile cosmetic emollient oils having a relatively high boiling point and function as a skin feel modifiers include, but are not hydrocarbons, fatty alcohols, fatty acids, non-volatile silicone oils, and esters such as glycerides and glycol esters.

Suitable ancillary oils include, but are not limited to isotridecyl isononanoate, isostearyl isostearate, isocetyl isosteatrate, isopropyl isostearate, isodecyl isonoanoate, cetyl octanoate, isononyl isononanoate, isocetyl myristate, isotridecyl myristate, isopropyl myristate, isostearyl palmitate, isocetyl palmitate, isodecyl palmitate, isopropyl palmitate, octyl palmitate, caprylic/capric acid triglyceride, glyceryl tri-2-ethylhexanoate, neopentyl glycol di(2-ethyl hexanoate), diisopropyl dimerate, tocopherol, tocopherol acetate, avocado oil, camellia oil, turtle oil, macadamia nut oil, corn oil, mink oil, olive oil, rapeseed oil, egg yolk oil, sesame oil, persic oil, wheat germ oil, pasanqua oil, castor oil, linseed oil, safflower oil, cotton seed oil, perillic oil, soybean oil, peanut oil, tea seed oil, kaya oil, rice bran oil, china paulownia oil, Japanese paulownia oil, jojoba oil, rice germ oil, glycerol trioctanate, glycerol triisopalmiatate, trimethylolpropane triisostearate, glycerol tri-2-ethylhexanoate, pentaerythritol tetra-2-ethylhexanoate, lanolin, liquid lanolin, liquid paraffin, squalane, vaseline, and mixtures thereof. Commercially available oils include, for example, tridecyl isononanoate with tradename Crodamol TN available from Croda, Hexalan available from Nisshin Seiyu, and tocopherol acetates available from Eisai.

Non-volatile cosmetic emollients may include waxes such as, but not limited to paraffin wax, microcrystalline wax, ozokerite wax, ceresin wax, carnauba wax, candelilla wax, and eicosanyl behenate.

Non-volatile silicon oils may be used including, but not limited to polymethylphenylsiloxane, polydiphenylsiloxane, polydiethylsiloxane, polydimethylsiloxane (dimethicone). For purposes of the present disclosure, a non-volatile silicon oil is defined as one that has a kinematic viscosity greater than 10 centiStokes (cSt).

Suitable ancillary oils include polyalkyl or polyaryl siloxanes as disclosed in U.S. 6,936,241.

Suitable ancillary oils useful herein include the various grades of mineral oils. Mineral oils are liquid mixtures of hydrocarbons that are obtained from petroleum. Specific examples of suitable hydrocarbons include paraffin oil, mineral oil, dodecane, isododecane, hexadecane, isohexadecane, eicosene, isoeicosene, tridecane, tetradecane, polybutene, polyisobutene, and mixtures thereof.

The non-volatile oil may not comprise a "volatile" silicone oil. A specifically excluded volatile silicone oil is decamethylcyclopentanasilaxane, commonly known as "D5."

In certain embodiments, the particulate substrate of the present invention is coated with a plurality of surface-active agents. Tables II and III disclose a kaolin substrate which is coated with three surface-active agents: triethoxycaprylylsilane, myristic acid, and stearoyl glutamate, where each surface-active agent is linked to the kaolin substrate by a polyvalent metal. Table IV discloses silica (comparative), mica, bismuth oxychloride, and aluminum calcium sodium silicate substrates, each coated with the same triple coating of surface-active agents. Any two or more surface-active agents may be combined as a plural coating. Plural coatings, and methods to apply them, are disclosed in co-pending applications 12/273,495 and 12/115,901.

The surface-active agents are chemically immobilized onto the surface of the powder material by the methods known in the art, such as those described in one or more of U.S. Patents 5,897,868; 6,482,441; 5,744,126; 7,374,783. Surface-active agents may be chemically immobilized by the methods described in one or more of co-pending U.S. Application, 11/142,468. Chemical immobilization differs from adding the surface-active agents to the powder material in that the treated powder material has a uniformly chemically bound reaction product.

The surface-active agents are present in an amount of at least 0.1% by weight, based on the weight of the powder material. Preferably, the surface-active agents are present in an amount ranging from about 1.0 to about 200% by weight; more preferably, from about 1.0 to about 60% by weight; and most preferably, from about 2.0 to about 30% by weight.

The inventors have unexpectedly shown that cosmetic powders potentiate the UV-activity of conventional organic UV-actives. Potentiation means that a given level of UV protection may be achieved at a far lower concentration of organic UV-active than is possible in the absence of cosmetic powder.

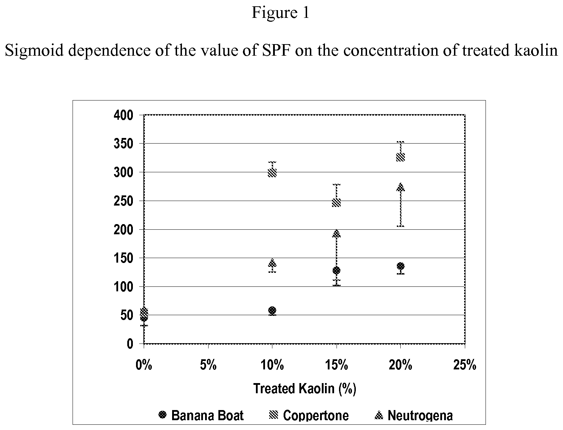

In Figure 1, the skin protection factor (SPF) yielded by three commercial sunblock formulations is compared in the presence and absence of increasing concentrations of a cosmetic powder. In Figure 1, the cosmetic powder is kaolin, the surface of which is coated with triethoxycaprylsilane - aluminum dimyristate - disodium stearoyl glutamate - aluminum hydroxide. The coating was applied by methods disclosed in U.S. Patents 7,276,113 and 6,482,441 and in U.S. Patent Application Publications 2006/0286048, 2008/0299158, and 2009/0191139. In the absence of cosmetic powder, each of the commercial formulations yields an SPF of approximately 50. The presence of cosmetic powder causes an increase in the observed SPF. Moreover, the observed SPF increases with increasing powder concentrations (each point is plotted as the mean ± SD, n = 3).

Unexpectedly, the observed SPF does not increase linearly with increasing powder concentrations. Low powder concentrations had little apparent affect on observed SPF values. At high powder concentrations, the process responsible for the increase in SPF appears to saturate at a limiting observed value. Each of the three commercial formulations appeared to give a unique saturation, or "plateau," value. At intermediate concentrations, the increase in observed SPF appears to follow sigmoid kinetics with an apparent inflection concentration which appears to be unique to each formulation (■, 7.5%; ▲, off scale; •, 12%). Neither a plateau value, nor an inflection in the process, is predictable from the prior art.

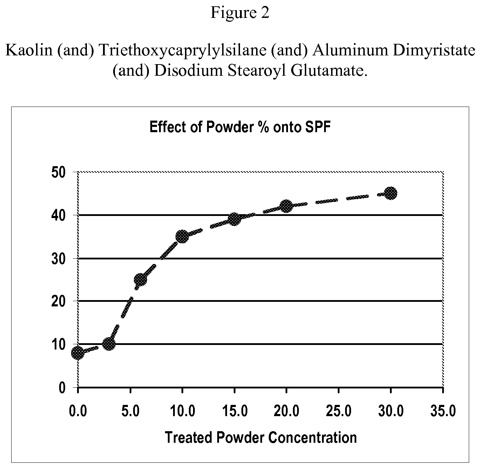

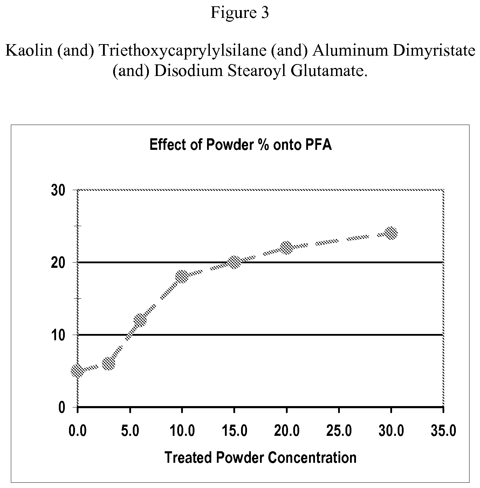

The sigmoid response of SPF (or PFA) to increasing concentrations of surface-treated powders is more clearly shown in Tables II, III, and IV, and Figures 2 and 3. In Tables II and III, an oil-in-water emulsion system is formulated with increasing amounts of kaolin the surface of which is treated with triethoxycaprylylsilane (and) aluminum myristate (and) disodium stearoyl glutamate. The data show that SPF (or PFA) increases with increasing powder concentration. Moreover, the increase in sun protection is not a linear function of powder concentration. A sigmoid curve is obtained where SPF (Figure 2) or PFA (Figure 3) is plotted as a function of powder concentration.

Table IV shows that the sigmoid response of SPF (or PFA) to increasing concentrations of surface-treated powders does not depend on the presence of a particular substrate. The SPF response to coated powders is demonstrated for silica (comparative), bismuth oxychloride, mica, and aluminum calcium sodium silicate substrates. Moreover, a plural coating is not required as Table IV demonstrates for talc coated with methicone or dimethicone and for kaolin coated with triethoxycaprylylsilane.

The ability of surface treated powders to enhance the SPF of a given oil-in-water (O/W) emulsion is not limited to a particular substrate or to a particular surface-treatment. Table IV demonstrates SPF enhancement where the substrate powder is silica (comparative), bismuth oxychloride, mica, aluminum calcium sodium silicate, talc, or kaolin powders, coated with any of triethoxycaprylylsilane, methicone, dimethicone, or triethoxycaprylylsilane (and) aluminum myristate (and) disodium stearoyl glutamate.

The powder-dependent, SPF-enhancement effect is not limited to O/W systems. Table V demonstrates powder-dependent SPF enhancement for a water-in-oil (W/O) system.

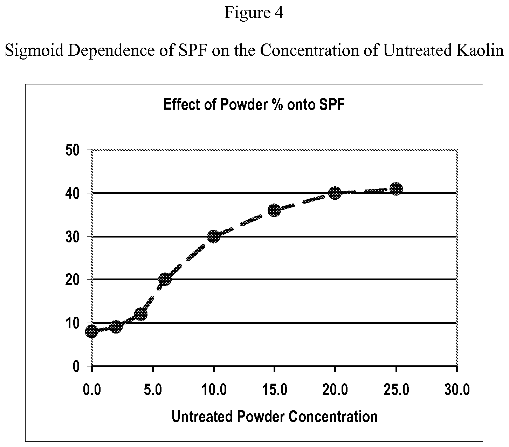

The ability of cosmetic powder materials to provide an SPF-enhancement is not limited to surface-treated powders. Table VI shows the effect of increasing concentrations of untreated kaolin (comparative) on the SPF of an O/W emulsion similar to the one described in Table II. Figures 4 (SPF) and 5 (PFA) demonstrate that untreated kaolin increases the SPF of O/W systems. Moreover, the concentration dependence is sigmoid with an inflection point.

As shown in Figure 1, the SPF observed for a given organic UV-active formulation depends in a sigmoid manner on the cosmetic powder concentration. The greatest concentration dependence is observed in the vicinity of the inflection point. Figure 1 further shows that for a given cosmetic powder the position of the inflection depends on the composition of the organic UV-active. At concentrations above the inflection concentration, the SPF rises until a saturation ("plateau") concentration is reached.

An aspect of the disclosure provides a desired SPF in the presence of lower organic UV-active concentrations by including at least one cosmetic powder at a concentration about or above an inflection concentration.

Figure 1 further shows that at concentrations above the inflection concentration, the SPF rises until a saturation concentration is reached. Moreover, the saturation concentration for a given cosmetic powder depends on the nature of the organic UV-active. An aspect of the disclosure includes at least one cosmetic powder included at a concentration up to approximately a saturation concentration.

Figure 1 demonstrates that surface-treated kaolin potentiates the SPF activity of the organic UV-active, but is not, itself, the UV-active agent. If UV-activity were a property of the treated kaolin, the titration curves in the presence of Banana Boat®, Coppertone®, and Neutrogena® should each be the same. However, the curves differ greatly. Thus showing that the treated kaolin is differentially potentiating the effect of the various UV-actives mixtures represented by these three commercial formulations.

**Table I:**

| Exemplary Formulation | | | |
|---|---|---|---|
| | | **Example** | **Comparative Example** |
| OIL Phase | Octinoxate (PARSOL OMC)* 1 | 2.00 | 2.00 |
| | Octocrylene (PARSOL 340)*1 | 2.00 | 2.00 |
| | Avobenzone (PARSOL 1789)*1 | 2.00 | 2.00 |
| | Isopropyl Myristate (IPM) | 2.00 | 2.00 |
| WATER Phase | DI Water | 70.00 | 80.00 |
| | Acrylates/C10-30 Alkyl Acrylate Crosscopolymer (Pemulen TR-1) | 0.15 | 0.15 |
| | Carbomer (Ultrez 10) | 0.25 | 0.25 |
| | Butylene Glycol | 4.00 | 4.00 |
| | Na₂ EDTA | 0.10 | 0.10 |
| | TWEEN 20 | 0.50 | 0.50 |
| | Kaolin (and) Triethoxycaprylylsilane (and) Aluminum Dimyristate (and) Disodium Stearoyl Glutamate | 10.00 | - |
| | 18% NaOH aq. soln. | 1.00 | 1.00 |
| | Phenoxy ethanol | 1.00 | 1.00 |
| | Acrylates Copolymer (Avalure AC118) | 5.00 | 5.00 |
| Total | | 100.00 | 100.00 |
| *in vitro* SPF | | 35 ± 6 | 12 ± 2 |
| *in vitro* PFA | | 21.6 | 8.3 |
| *in vitro* UVA/UVB | | 0.773 | 0.769 |
| *in vitro* SPF Index | | 5.8 | 2 |

Table I presents a non-limiting, exemplary formulation. The water and oil phases were formulated separately and then mixed.

An aqueous phase was formulated by loading deionized water into a high shear disperser mixer with acrylates (Pemulen® TR-1), carbomer (Ultrez® 10), and disodium EDTA. The mixture was blended at ∼1400 rpm with heating to ∼75°C. Upon dissolution of the solid materials, butylene glycol and Tween® 20 were added. The aqueous phase was completed by the dispersion of surface-treated kaolin.

An oil phase was separately formulated by heating the Octinoxate, octocrylene, avobenzone, and isopropyl myristate to ∼75°C.

The oil phase was added to the aqueous phase and emulsified by blending at ∼1400 rpm. The mixing blade was then changed from a disper to a propeller mixer. A sodium hydroxide solution was added to thicken the emulsion. Finally, phenoxyethanol and the acrylic copolymer (Avalure® AC118) were blended into the emulsion which was allowed to cool to room temperature with continued mixing.

The comparative example is an otherwise identical formulation with an additional portion of water in place of the treated kaolin. The formulation according to the invention yielded approximately three times the SPF of the comparative example.

This invention has industrial applicability in providing cosmetic formulations that provide high levels of SPF protection with low levels of organic, UV-actives.

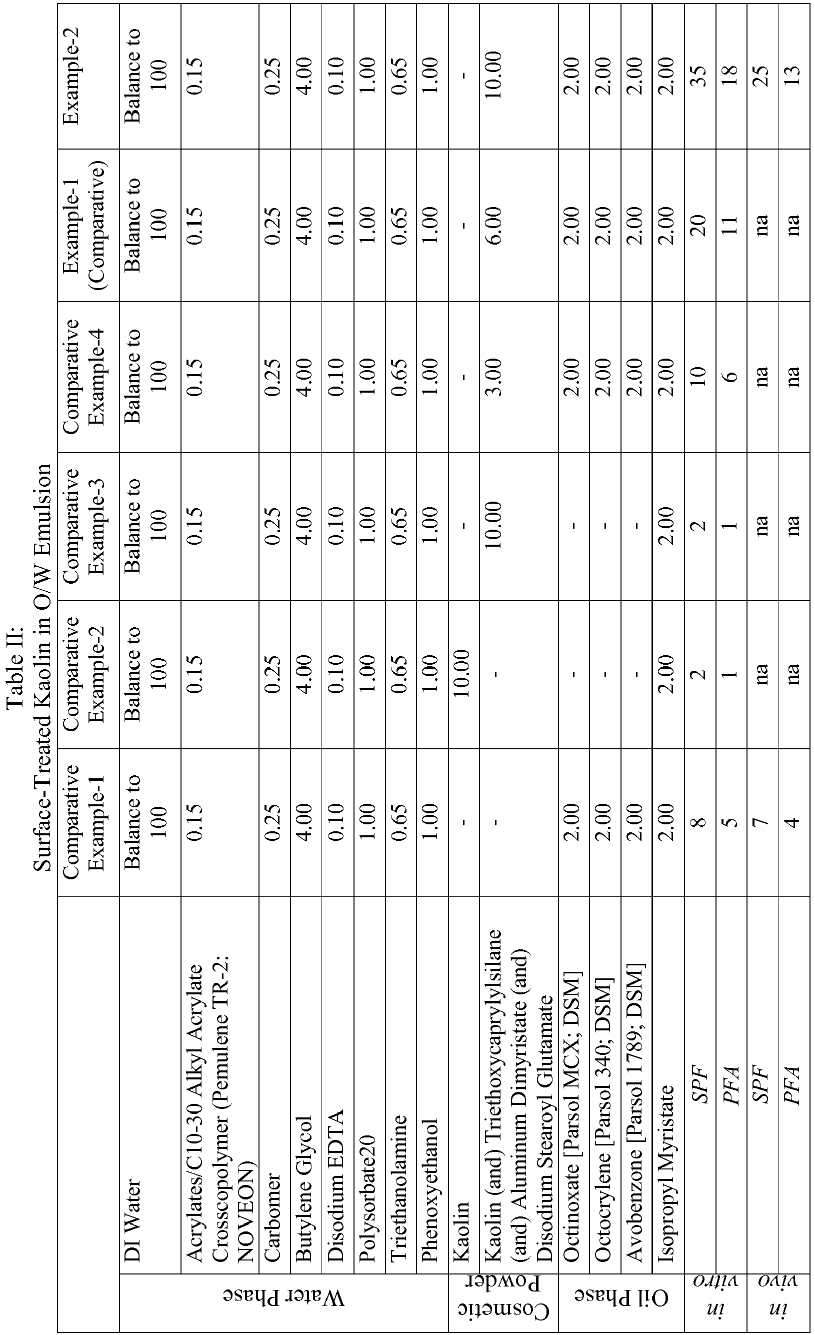

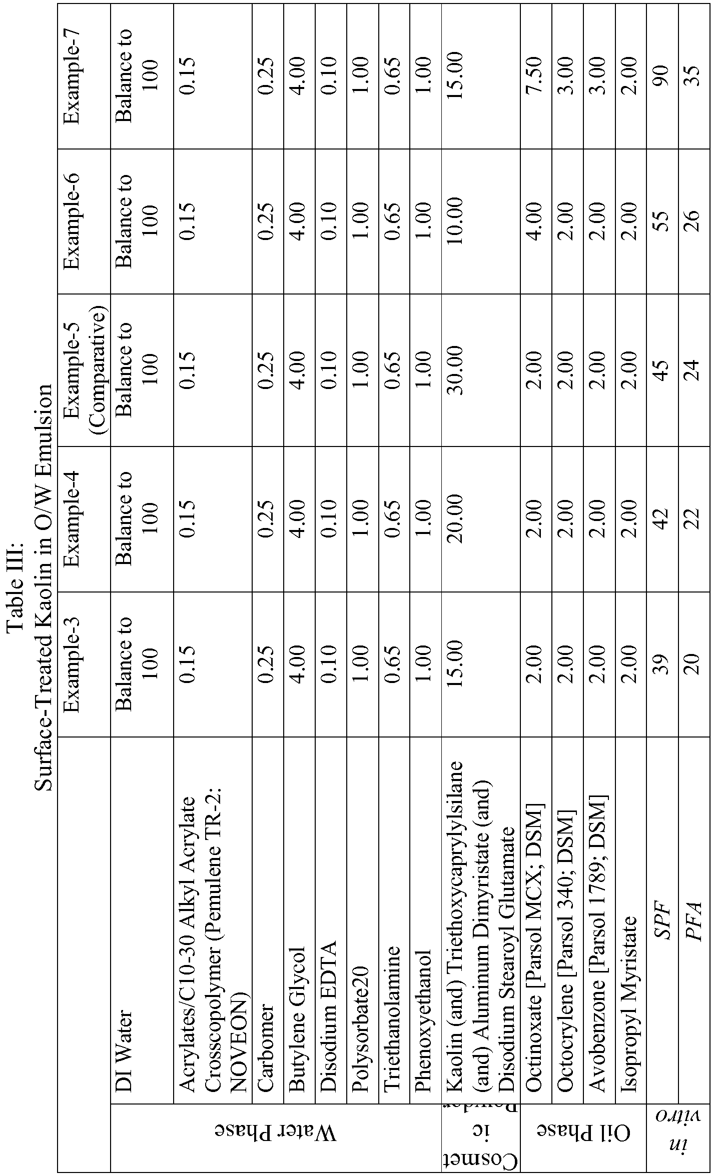

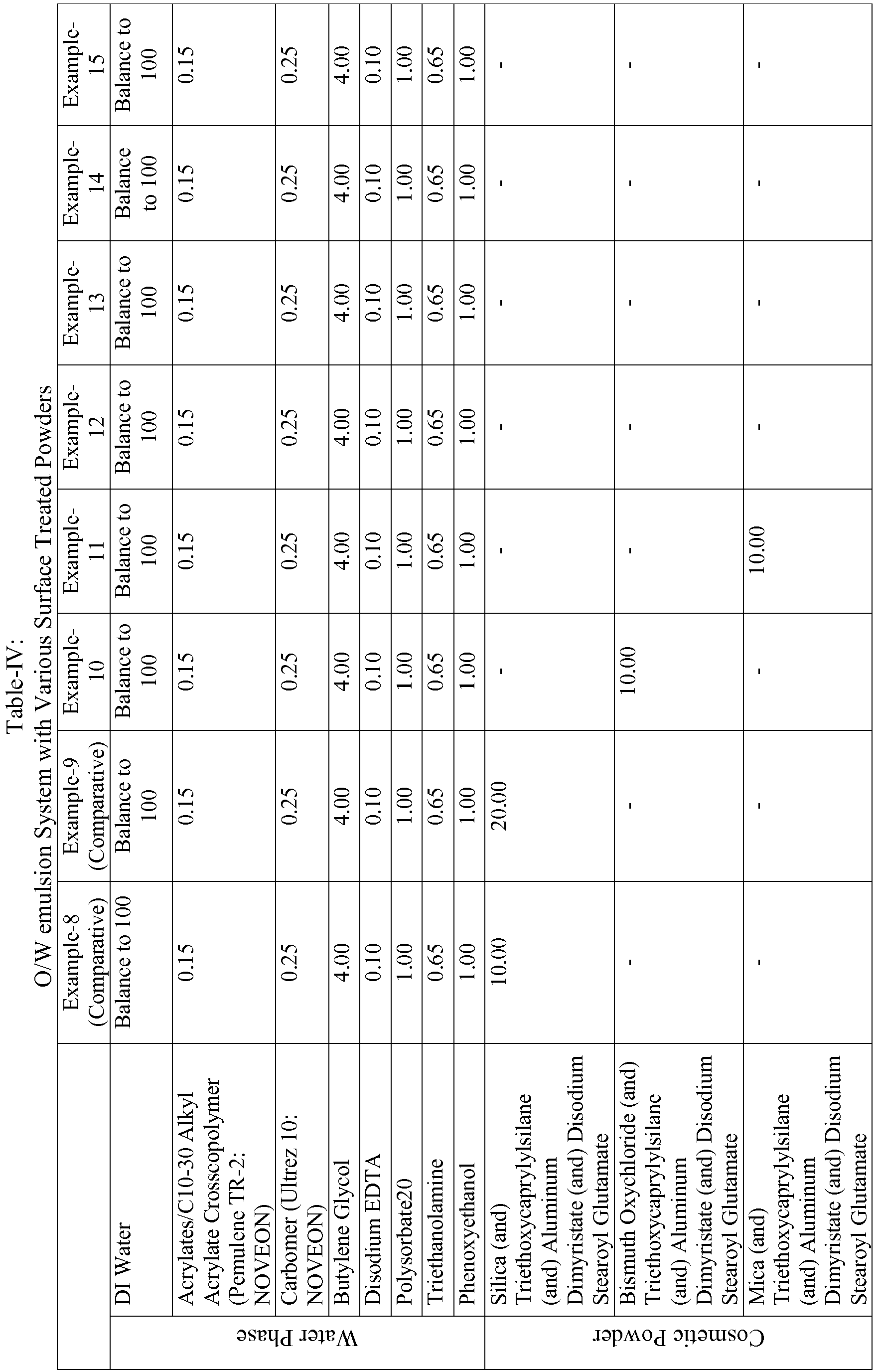

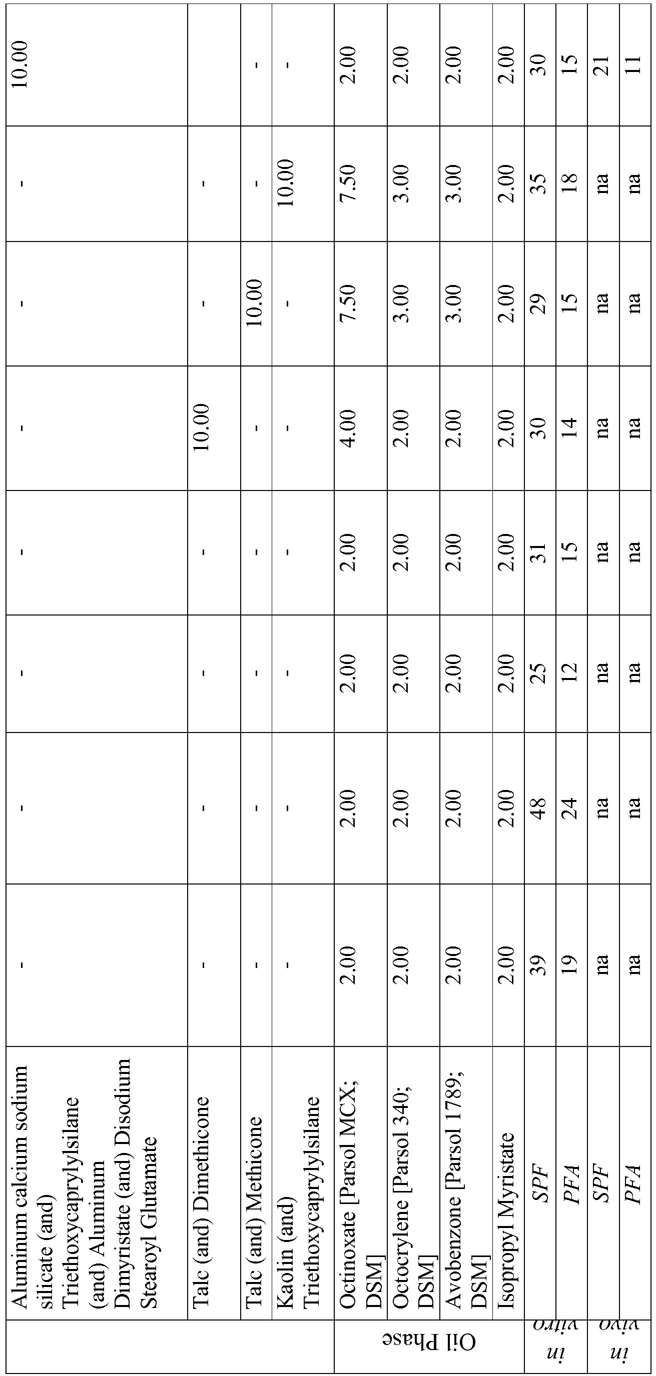

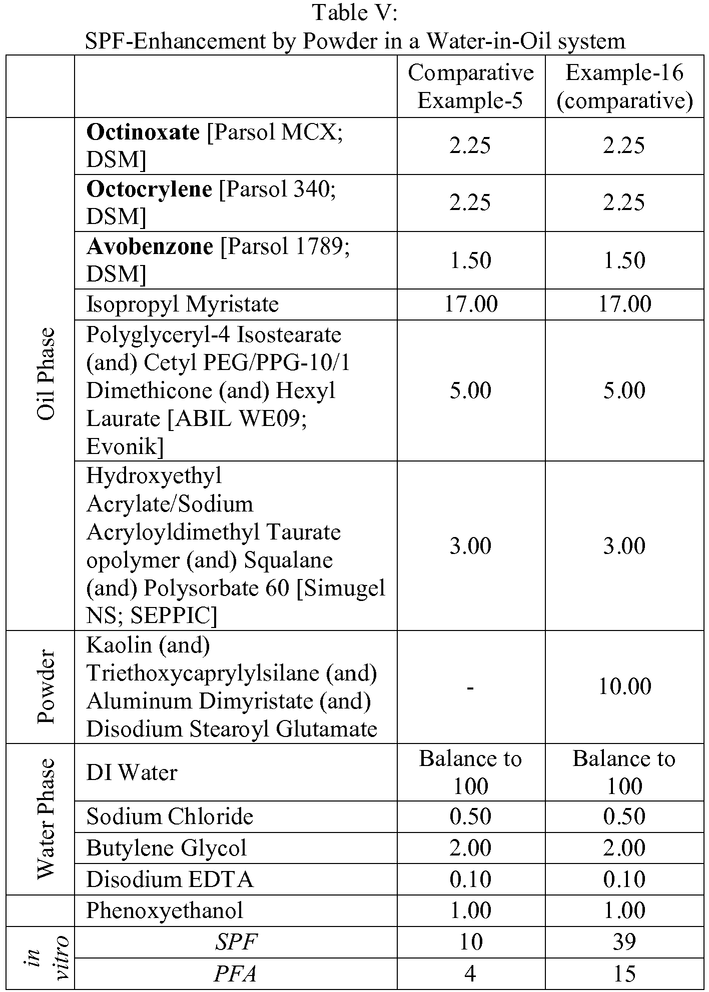

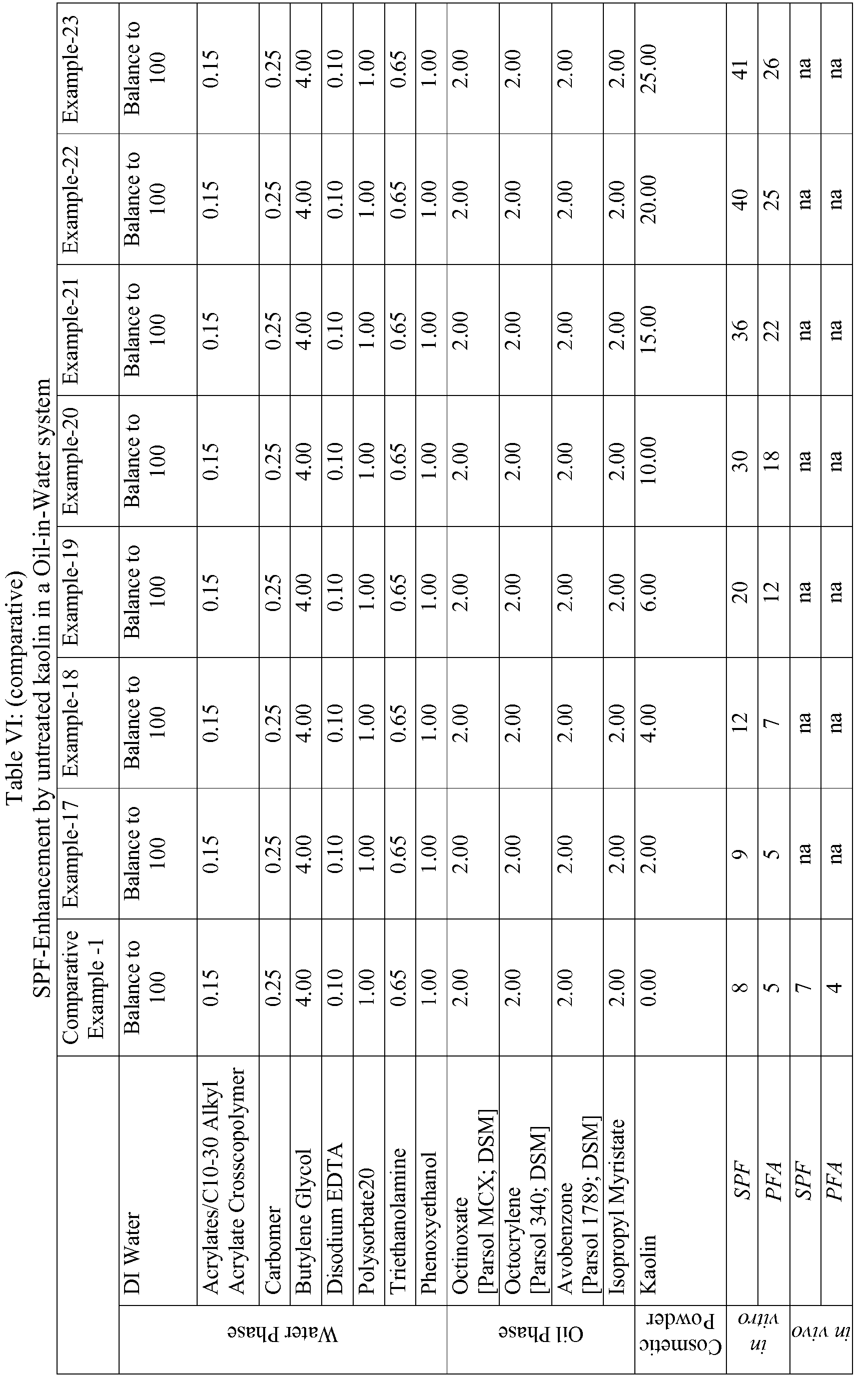

## Claims

1. An SPF-boosting composition having an SPF-index of at least 3.0, comprising organic UV-protective agents and a cosmetic powder, wherein a given level of UV protection is achieved at a lower concentration of organic UV-protective agents than is possible in the absence of the cosmetic powder, the composition comprising:
up to 30 wt% of a non-volatile oil comprising at least two organic UV-active materials,
at least 30 wt% of an aqueous phase; and
from 10 wt% to 25 wt% of the cosmetic powder, wherein said cosmetic powder is coated with at least one surface-active agent chemically-immobilized to a surface thereof, wherein the cosmetic powder selected from the group consisting of mica, aluminum calcium sodium silicate, talc, kaolin, bismuth oxychloride and mixtures thereof; and
wherein the at least one surface active agent is selected from any of triethoxycaprylylsilane, methicone, dimethicone, or triethoxycaprylylsilane (and) aluminum myristate (and) disodium stearoyl glutamate.

2. The SPF-boosting composition of Claim 1, wherein the at least two organic UV-active materials are selected from the group consisting of octinoxate, octocrylene, avobenzone, PABA, octyldimethyl-PABA, Phenylbenzimidazole sulfonic acid, Cinoxate, Dioxybenzone (Benzophenone-8), Oxybenzone (Benzophenone-3), Homosalate, Menthyl anthranilate, Octisalate, Sulisobenzone, Trolamine salicylate, Terephthalylidene Dicamphor Sulfonic Acid, 4-Methylbenzylidene camphor, Methylene Bis-Benzotriazolyl Tetramethylbutylphenol, Bis-ethylhexyloxyphenol methoxyphenol triazine, bisimidazylate, Drometrizole Trisiloxane, Octyl triazone, Diethylamino Hydroxybenzoyl Hexyl Benzoate, Iscotrizinol, Polysilicone-15, Amiloxate, Ethylhexyl Dimethoxybenzylidene Dioxoimidazolidine Propionate, and mixtures thereof.

3. The SPF-boosting composition of Claim 1, wherein said non-volatile oil further comprises an ancillary selected from the group consisting of oils and waxes.

4. The SPF-boosting composition of Claim 3, wherein said ancillary oil is selected from the group consisting of isopropyl myristate, isotridecyl isononanoate, isostearyl isostearate, isocetyl isostearate, isopropyl isostearate, isodecyl isononanoate, cetyl octanoate, isononyl isononanoate, isopropyl myristate, isocetyl myristate, isotridecyl myristate, isostearyl palmitate, isocetyl palmitate, isodecyl palmitate, isopropyl palmitate, octyl palmitate, caprylic/ capric triglyceride, glyceryl tri-2-ethylhexanoate, neopentyl glycol di(2-ethylhexanoate), diisopropyl dimerate, tocopherol, tocopherol acetate, avocado oil, camellia oil, turtle oil, macadamia nut oil, corn oil, mink oil, olive oil, grapeseed oil, egg yolk oil, sesame oil, wheat germ oil, castor oil, linseed oil, safflower oil, cotton seed oil, soybean oil, peanut oil, tea seed oil, rice bran oil, rice germ oil, jojoba oil, glycerol trioctanoate, glycerol triisopalmitate, trimethylolpropane triisostearate, pentaerythritol tetra-2-ethylhexanoate, lanolin, liquid lanolin, liquid paraffin, squalane, squalane, vaseline, and mixtures thereof, or said ancillary wax is selected from the group consisting of paraffin wax, microcrystalline wax, ozokerite wax, ceresin wax, carnauba wax, candelilla wax, eicosanyl behenate, and mixtures thereof.

5. The SPF-boosting composition of Claim 3, wherein said ancillary is a silicon oil selected from the group consisting of polymethylphenylsiloxane, polydiphenylsiloxane, polydiethylsiloxane, polydimethylsiloxane (dimethicone) more than 10 cSt and mixtures thereof.

6. The SPF-boosting composition of Claim 1, wherein said composition has an SPF-index of at least 4.0, more preferably of at least 5.0.

7. The SPF-boosting composition of Claim 1, wherein said composition is free of volatile silicone oil.

8. The SPF-boosting composition of Claim 1, wherein said cosmetic powder is coated with a plurality of surface-active agents chemically-immobilized to a surface thereof.

## Patentansprüche

1. SPF-verstärkende Zusammensetzung, die einen SPF-Index von mindestens 3,0 aufweist, umfassend organische UV-Schutzmittel und einen Kosmetikpuder, wobei ein gegebener Grad von UV-Schutz bei einer niedrigeren Konzentration an organischen UV-Schutzmitteln erreicht wird, als es bei Fehlen des Kosmetikpuders möglich ist, die Zusammensetzung umfassend:
bis zu 30 Gew.-% eines nichtflüchtigen Öls, das mindestens zwei organische UV-aktive Materialien umfasst,
mindestens 30 Gew.-% einer wässrigen Phase; und
von 10 Gew-% bis 25 Gew-% des Kosmetikpuders, wobei der Kosmetikpuder mit mindestens einem oberflächenaktiven Mittel beschichtet ist, das auf einer Oberfläche desselben chemisch immobilisiert ist, wobei der Kosmetikpuder aus der Gruppe bestehend aus Glimmer, Aluminium-Calcium-Natriumsilicat, Talkum, Kaolin, Bismutchloridoxid und Gemischen aus diesen ausgewählt ist; und
wobei mindestens ein oberflächenaktives Mittel aus der Gruppe aus beliebigen von Triethoxycaprylylsilan, Methicon, Dimethicon oder Triethoxycaprylylsilan (und) Aluminiummyristat (und) Dinatriumstearoylglutamat ausgewählt ist.

2. SPF-verstärkende Zusammensetzung nach Anspruch 1, wobei die mindestens zwei UV-aktiven Materialien aus der Gruppe, bestehend aus Octinoxat, Octocrylen, Avobenzon, PABA, Octyldimethyl-PABA, Phenylbenzimidazolsulfonsäure, Cinoxat, Dioxybenzon (Benzophenon-8), Oxybenzon (Benzophenon-3), Homosalat, Menthylanthranilat, Octisalat, Sulisobenzon, Trolaminsalicylat, Terephthalylidendicamphorsulfonsäure, 4-Methylbenzylidencamphor, Methylen-Bis-Benzotriazolyl-Tetramethylbutylphenol, Bis-Ethylhexyloxyphenolmethoxyphenoltriazin, Bisimidazylat, Drometrizoltrisiloxan, Octyltriazon, Diethylaminohydroxybenzoylhexylbenzoat, Iscotrizinol, Polysilicon-15, Amiloxat, Ethylhexyldimethoxybenzylidendioxoimidazolidinpropiona t und Gemischen aus diesen ausgewählt sind.

3. LSF-verstärkende Zusammensetzung nach Anspruch 1, wobei das nichtflüchtige Öl ferner einen Zusatzstoff enthält, der aus der Gruppe bestehend aus Ölen und Wachsen ausgewählt ist.

4. SPF-verstärkende Zusammensetzung nach Anspruch 3, wobei das Zusatzöl aus der Gruppe bestehend aus Isopropylmyristat, Isotridecylisononanoat, Isostearylisostearat, Isocetylisostearat, Isopropylisostearat, Isodecylisononanoat, Cetyloctanoat, Isononylisononanoat, Isopropylmyristat, Isocetylmyristat, Isotridecylmyristat, Isostearylpalmitat, Isocetylpalmitat, Isodecylpalmitat, Isopropylpalmitat, Octylpalmitat, Capryl-/ Caprintriglycerid, Glyceryl-tri-2-ethylhexanoat, Neopentylglycol-di(2-Ethylhexanoat), Diisopropyldimerat, Tocopherol, Tocopherolacetat, Avocadoöl, Kamelienöl, Schildkrötenöl, Macadamianussöl, Maisöl, Nerzöl, Olivenöl, Traubenkernöl, Eigelböl, Sesamöl, Weizenkeimöl, Rizinusöl, Leinöl, Safloröl, Baumwollsamenöl, Sojaöl, Erdnussöl, Teesamenöl, Reisöl, Reiskeimöl, Jojobaöl, Glycerintrioctanoat, Glycerintriisopalmitat, Trimethylolpropantriisostearat, Pentaerythritol-tetra-2-ethylhexanoat, Lanolin, flüssiges Lanolin, flüssiges Paraffin, Squalan, Squalan, Vaseline und Gemischen aus diesen ausgewählt ist, oder das Zusatzwachs aus der Gruppe bestehend aus Paraffinwachs, mikrokrystallinem Wachs, Ozokeritwachs, Ceresinwachs, Carnaubawachs, Candelillawachs, Eicosanylbehenat und Gemischen aus diesen ausgewählt ist.

5. SPF-verstärkende Zusammensetzung nach Anspruch 3, wobei der Hilfsstoff ein Silikonöl ist, der aus der Gruppe bestehend aus Polymethylphenylsiloxan, Polydiphenylsiloxan, Polydiethylsiloxan, Polydimethylsiloxan (Dimethicon) mehr als 10 cSt und Gemischen aus diesen ausgewählt ist.

6. LSF-verstärkende Zusammensetzung nach Anspruch 1, wobei der Zusammensetzung einen SPF-Index von mindestens 4,0, besonders bevorzugt von mindestens 5,0 aufweist.

7. SPF-verstärkende Zusammensetzung nach Anspruch 1, wobei die Zusammensetzung frei von flüchtigen Silikonölen ist.

8. SPF-verstärkende Zusammensetzung nach Anspruch 1, wobei der Kosmetikpuder mit mehreren oberflächenaktiven Mitteln beschichtet ist, die auf einer Oberfläche desselben chemisch immobilisiert sind.

## Revendications

1. Composition pour améliorer le facteur de protection solaire (SPF) ayant un indice SPF d'au moins 3,0, comprenant des agents organiques de protection contre les UV et une poudre cosmétique, dans laquelle un niveau donné de protection contre les UV est obtenu à une concentration d'agents organiques de protection contre les UV inférieure à ce qui est possible en l'absence de la poudre cosmétique, la composition comprenant :
jusqu'à 30 % en poids d'une huile non volatile comprenant au moins deux matières organiques actives aux UV,
au moins 30 % en poids d'une phase aqueuse ; et
de 10 % en poids à 25 % en poids de la poudre cosmétique, ladite poudre cosmétique étant enrobée par au moins un agent tensio-actif immobilisé chimiquement sur une surface de celle-ci, la poudre cosmétique étant choisie dans le groupe consistant en le mica, le silicate d'aluminium, de calcium et sodium, le talc, le kaolin, l'oxychlorure de bismuth et leurs mélanges ; et
dans laquelle ledit au moins un agent tensio-actif est choisi parmi l'un quelconque du triéthoxycaprylylsilane, de la méthicone, de la diméthicone ou du triéthoxycaprylylsilane (et) du myristate d'aluminium (et) du glutamate de stéaroyle disodique.

2. Composition pour améliorer le SPF selon la revendication 1, dans laquelle lesdites au moins deux matières organiques actives UV sont choisies dans le groupe consistant en l'octinoxate, l'octocrylène, l'avobenzone, PABA, l'octyldiméthyl-PABA, l'Acide phénylbenzimidazole sulfonique, le Cinoxate, la Dioxybenzone (Benzophénone-8), l'Oxybenzone (Benzophénone-3), l'Homosalate, l'Anthranilate de menthyle, l'Octisalate, la Sulisobenzone, le Salicylate de trolamine, l'Acide Téréphtalylidène Dicamphre Sulfonique, le 4-Méthylbenzylidène camphre, le Méthylène Bis-Benzotriazolyl Tétraméthylbutylphénol, la Bis-éthylhexyloxyphénol méthoxyphénol triazine, le bisimidazylate, le Drométrizole Trisiloxane, l'Octyl triazone, le Diéthylamino Hydroxybenzoyl Hexyl Benzoate, l'Iscotrizinol, la Polysilicone-15, l'Amiloxate, l'Ethylhexyl Diméthoxybenzylidène Dioxoimidazolidine Propionate et leurs mélanges.

3. Composition pour améliorer le SPF selon la revendication 1, dans laquelle ladite huile non volatile comprend en outre un auxiliaire choisi dans le groupe consistant en les huiles et les cires.

4. Composition pour améliorer le SPF selon la revendication 3, dans laquelle ladite huile auxiliaire est choisie dans le groupe consistant en le myristate d'isopropyle, l'isononanoate d'isotridécyle, l'isostéarate d'isostéaryle, l'isostéarate d'isocétyle, l'isostéarate d'isopropyle, l'isononanoate d'isodécyle, l'octanoate de cétyle, l'isononanoate d'isononyle, le myristate d'isopropyle, le myristate d'isocétyle, le myristate d'isotridécyle, le palmitate d'isostéaryle, le palmitate d'isocétyle, le palmitate d'isodécyle, le palmitate d'isopropyle, le palmitate d'octyle, le triglycéride caprylique/caprique, le tri-2-éthylhexanoate de glycéryle, le di(2-éthylhexanoate) de néopentyl glycol, le dimérate de diisopropyle, le tocophérol, l'acétate de tocophérol, l'huile d'avocat, l'huile de camélia, l'huile de tortue, l'huile de noix de macadamia, l'huile de maïs, l'huile de vison, l'huile d'olive, l'huile de pépins de raisin, l'huile de jaune d'oeuf, l'huile de sésame, l'huile de germes de blé, l'huile de ricin, l'huile de lin, l'huile de carthame, l'huile de coton, l'huile de soja, l'huile d'arachide, l'huile de graines de thé, l'huile de son de riz, l'huile de germes de riz, l'huile de jojoba, le trioctanoate de glycérol, le triisopalmitate de glycérol, le triisostéarate de triméthylolpropane, le tétra-2-éthylhexanoate de pentaérythritol, la lanoline, la lanoline liquide, la paraffine liquide, le squalane, le squalane, la vaseline et leurs mélanges, ou ladite cire auxiliaire est choisie dans le groupe consistant en la cire de paraffine, la cire microcristalline, la cire d'ozokérite, la cire de cérésine, la cire de carnauba, la cire de candelilla, le béhénate d'eicosanyle et leurs mélanges.

5. Composition pour améliorer le SPF selon la revendication 3, dans laquelle ledit auxiliaire est une huile de silicone choisie dans le groupe consistant en le polyméthylphénylsiloxane, le polydiphénylsiloxane, le polydiéthylsiloxane, le polydiméthylsiloxane (diméthicone) de plus de 10 cSt et leurs mélanges.

6. Composition pour améliorer le SPF selon la revendication 1, dans laquelle ladite composition a un indice SPF d'au moins 4,0, de façon davantage préférée d'au moins 5,0.

7. Composition pour améliorer le SPF selon la revendication 1, dans laquelle ladite composition est exempte d'huile de silicone volatile.

8. Composition pour améliorer le SPF selon la revendication 1, dans laquelle ladite poudre cosmétique est enrobée par une pluralité d'agents tensio-actifs immobilisés chimiquement sur une surface de celle-ci.
